# EUROPEAN PATENT SPECIFICATION

(11) **EP 3 972 656 B1**
(45) Date of publication and mention of the grant of the patent: **14.01.2026**
(21) Application number: 20730734.9
(22) Date of filing: 18.05.2020
(51) Int. Cl.: A61L 15/18, A61F 13/84, A61L 15/42, A61L 15/62, A61L 15/46, A61F 13/15

(54) **ABSORBENT ARTICLE WITH SYSTEM FOR AGGREGATING AND ISOLATING FECES**
ABSORBIERENDER ARTIKEL MIT SYSTEM ZUR AGGREGATION UND ISOLIERUNG VON FÄKALIEN
ARTICLE ABSORBANT AVEC SYSTÈME D'AGRÉGATION ET D'ISOLATION DES MATIÈRES FÉCALES

(30) Priority: 20.05.2019 IT 201900007006
(43) Date of publication of application: 30.03.2022
(73) Proprietor: Biodiapers S.r.l., 73024 Maglie (LE) (IT)
(72) Inventor: CHIARELLI, Piero, 73024 Maglie (LE) (IT); SURACE, Filippo, 73024 Maglie (LE) (IT)
(74) Representative: Fiammenghi, Eva
(86) International application number: PCT/IB2020/054686
(87) International publication number: WO 2020/234745

(56) References cited:
- US-A- 5 998 695
- US-B1- 6 395 955
- US-B1- 7 763 003

## Description

### Field of the art

The present invention refers to the field of absorbent articles such as diapers, pads/tampons and the like. More in detail the present description refers to a particular and innovative absorbent article and especially to a diaper for children or adults with problems of incontinence in general, which in addition to the common characteristics of diapers currently available on the market regarding the capacity to absorb urine, also has a suitable system for the absorption of feces and for their isolation within the structure of the diaper itself. All this with the goal of avoiding the contact of the feces with the skin of the wearer of the absorbent article and the possibility of onset of infecti ons.

### State of the art

Underwear garments with absorbent properties adapted to be worn by newborn babies and children from birth to about two years old, and also by elderly people with problems of incontinence who are unable to control their own intestinal and urinary physiological needs, have for many years been known with the name diapers. From a structural standpoint, this is typically a disposable product, commonly comprising three layers: the first layer is the most internal which comes into direct contact with the skin, typically of the child. The characteristics of this layer include softness and the sensation that it must confer even following the presence of urine. The composition of this layer is among the most important since this comes into contact with the skin of the wearer. The external layer, impermeable, instead has the task of not allowing the physiological liquid - typically urine - to exit outward, and for such purpose is made of plastic material. Some producers of diapers and absorbent articles, for personal/genital hygiene in general, make products with lower environmental impact by using a plant-based plastic, specifically based on polylactic acid.

The internal layer has the function of absorbing "the wet/moisture" and retaining it even for many hours without outflows thereof occurring. In order to ensure that the wet/moisture does not exit outward with the movements of the child, all disposable diapers presently on the market introduce, within a cellulose shell (or shell made of fibers with similar properties such as those based on wheat or corn), super absorbent chemical gels (SAPs), such as sodium polyacrylate capable of absorbing considerably quantities of the physiological liquids of interest. SAPs are relatively new materials since they first appeared in the early 1970s, and they had widespread commercial diffusion in the 1980s. The tests regarding the effects that these substances can have on the health of the individual over the long term cannot be considered terminated today, even if the present results pertaining to their potential toxicity indicate safety guarantees for their use and lack of danger. At any rate, it is known that the SAPs are substances derived from petroleum that can therefore contain chemical substances which are potentially compromising for the health of the individual.

In addition to the aspects tied to the health-impacting consequences of the constituent materials which are undoubtedly today the object of study and incentivizing innovation in the field, these articles can have further limitations due to their effectiveness and in particular due to their performances in protecting the skin from the feces possibly contained in the absorbent article. Indeed it is recognized that absorbent articles such as diapers are generally designed for having a satisfactory functionality based only on the absorption of urine while the solid fractions, typically feces, remain on the surface of the absorbent article, thus continually coming into contact with the skin of the wearer of the article, up until the latter is removed and changed. All of the above indicates that acceptable hygienic conditions are not ensured.

For the purpose of comprehension of the present invention, described in detail hereinbelow, it is of interest to present that - following experimental tests - the inventors observed that diapers, as well as the pads/tampons that can be presently found on the market, absorb the fluids (urine and other fluid and aqueous parts present in the feces) via impregnation, thus leaving the surrounding environment moist and creating conditions that facilitate bacterial proliferation, clearly harmful for health. This present aspect is substantially due to the complete lack of a system for treating, and especially for separating, feces from urine. It follows that the agglomerates of feces can remain in contact with the skin, thus exposing it to the presence of highly aggressive and harmful bacteria and enzymes, thus even being able to cause serious problems and diseases.

In fact it must be considered that for the production of this type of absorbent articles, as they appear on the market today, gel-based natural or synthetic fibrous materials have always been used which have the capacity to absorb aqueous fluids. Therefore, such products must inevitably be considered ineffective if not unsuitable for allowing the separation of feces from urine and simultaneously for ensuring that the skin, in contact with the organic physiological waste, is protected from the infection risk caused by bacterial proliferation.

Specifically, present-day diapers lack internal mechanisms which allow the separation of feces from urine. All this endows a sensation of discomfort for the wearer, and above all involves the risk of possible onset of irritations or infections of the skin in the genital areas.

Object of the present invention is therefore that of proposing a system, and specifically an absorbent article such as a diaper or the like, which is provided with a system for separating feces so as to prevent all of the abovementioned problems. The solution that the inventors offer for attaining said system provides for, as will be described in detail hereinbelow, the use of a known substance, and specifically of clay which - when suitably pretreated and housed in the absorbent article in a particular manner - is capable of "neutralizing" and isolating the feces possibly present in the diaper, thus protecting the skin from contact with the feces.

The use of clay as absorbent agent in the health/medical field is already known, since its absorbing and bonding properties due to its composition are known. Indeed this is an extremely fine non-lithified sediment mainly comprising aluminosilicate hydrates belonging to the phyllosilicates class. The minerals that constitute it are clay minerals such as diosmectite, montmorillonite, aluminum and magnesium silicates, kaolinite and sericite and they contribute to imparting the characteristics of plasticity, high bonding power and thickening power for suspensions containing high percentages of aqueous liquids.

The document US 3935363 describes an absorbent product containing flocculated clay mineral aggregates, so as to implement the properties of the absorbent product.

According to that described in said prior art document, it is provided that the clay minerals are pretreated with a flocculating agent such as, for example, small quantities of polymer materials such as polyacrylic acid, polystyrene and the like, so as to allow an optimal flocculation. Following the flocculation, the aggregates are filtered in order to be separated from the aqueous solution and subsequently dried in order to be incorporated in the fibrous support, thus attaining the absorbent product.

The solution presented by the invention, object of said prior art document, even if it is undoubtedly effective in improving the absorbent power of the clay minerals with respect to the fluids, has a drawback consisting of the need to give the clay compound a complete pretreatment before the use thereof in the form effective for the requested purpose. Such pretreatment signifies an industrial process for the preparation of clay floccules which inevitably has additional costs for its implementation. In addition, the absorbent articles provided with said pretreated clay component have in any case proven ineffective in isolating the skin from contact with the feces, in fact only being able to decrease the aqueous component of the feces and in any case exposing the skin to contact therewith and to potential infection risk.

The documents US 5 998 695 A and US 6 395 955 B1 disclose an absorbent article (1) adapted for absorbing and housing urine and human excrements, said absorbent article (1) being characterized in that it comprises, in its structure at the height of its central portion, a composition (5) with clay and thickening polymer base.

The document US5869033 also describes an absorbent article, and especially a diaper, capable of preventing the irritation of the skin due to the use of organophilic clay, i.e. clay specifically treated with organic molecules. Nevertheless, even such document does not confront the problem of separating the feces, thus allowing that the inevitable contact of the latter with the skin still exposes the wearer of the absorbent article to the risk of infection onset.

For such purpose, the object of the present industrial invention patent application is that of proposing an absorbent article such as, by way of a non-limiting example, a diaper which incorporates clay so as to eliminate all the fluid components of the feces and simultaneously which allows isolating the latter from the skin, thus avoiding, upstream, the infection risk. All this, as will be described in detail hereinbelow, due to a particular housing suitably designed in the structure of the absorbent article.

### Description of the invention

The present description refers to a particular absorbent article as defined in claim 1 and especially to a diaper (for children or elderly people or people with problems of incontinence in general) which, in addition to effectively completing all the functions carried out by the diapers presently available on the market, is capable of isolating the skin of the wearer from contact with feces, allowing the housing of the latter in a suitable compartment made in the structure of the absorbent article itself.

Still more in detail, the present description refers to a diaper which, due to the presence in its structure of a clay-based composition, is capable of "neutralizing" the feces, inhibiting the bacterial development and also re-establishing a more physiological pH therein. Said diaper is at any rate characterized by the presence of a further component which contributes to limiting, if not entirely eliminating, the possibility of onset of infection risk caused by the contact of feces with the skin of the wearer of the diaper.

Specifically, said absorbent article is provided with a tray-like housing made of self-expandable material which is incorporated in the absorbent article and into which the feces can access through an inlet with superimposed tabs present on the surface of the internal portion of the diaper, the portion which is typically in contact with the skin of the genital areas of the wearer.

Still more specifically, said diaper has a tray at its interior, on whose internal walls a film made of biocompatible polymer material is present which is adapted to coat the clay-based composition present in the tray. Upon contact with the feces, said film is dissolved, thus allowing the outflow of the clay, allowing the performance of the thickening and neutralizing action thereof with regard to the entering feces.

For the purpose of a more complete description, it is in the Applicant's interest to specify that the actual fecal consistency depends on the diet, age and physical conditions of the wearer of the absorbent article and typically of the child. The feces of breast-fed babies have fluid and pasty consistency, while those of artificially-fed babies children are denser and larger. The three most common cases of fecal consistency taken under consideration for describing the operation of the diaper according to the present invention regard:
- fluid feces, towards which the clay composition has thickening and compacting action for the solid portion, while the excess liquid fraction is absorbed by the fluff. All this allows the feces to remain isolated from the skin within the collection tray.
- pasty feces: in this case, the feces are compacted and hardened by the clay compound and maintained dry by the absorbent fluff, thus remaining isolated from the skin within the collection tray.
- solid feces: in this case, the effect of the clay composition is that of further hardening and compacting the feces that come to be isolated within said collection tray.

It is also in the interest of the Applicant to specify that among the various properties of clay in the particular context where it carries out its action according to that described above, there is that of sanitizing the affected environment.

Advantageously the sanitizing action of the clay in the absorbent article according to the present invention allows blocking the bacterial development.

Advantageously said anti-bacterial action also allows the re-establishment of a more physiological pH.

Advantageously said use of clay within the absorbent article is indicated in a system for protecting the hygiene and health of the individual.

Advantageously, in all embodiments thereof, in addition to offering the aforesaid advantages, the use of clay, together with its particular placement within the tray of the absorbent article according to the present invention, allows ensuring a certain and more consistent level of local cleaning of the skin of the wearer with respect to that obtainable with the more modern diaper models that can be currently found on the market.

### Description of the figures

The invention, object of the present description, will be described in detail hereinbelow with reference to the enclosed figures in which:

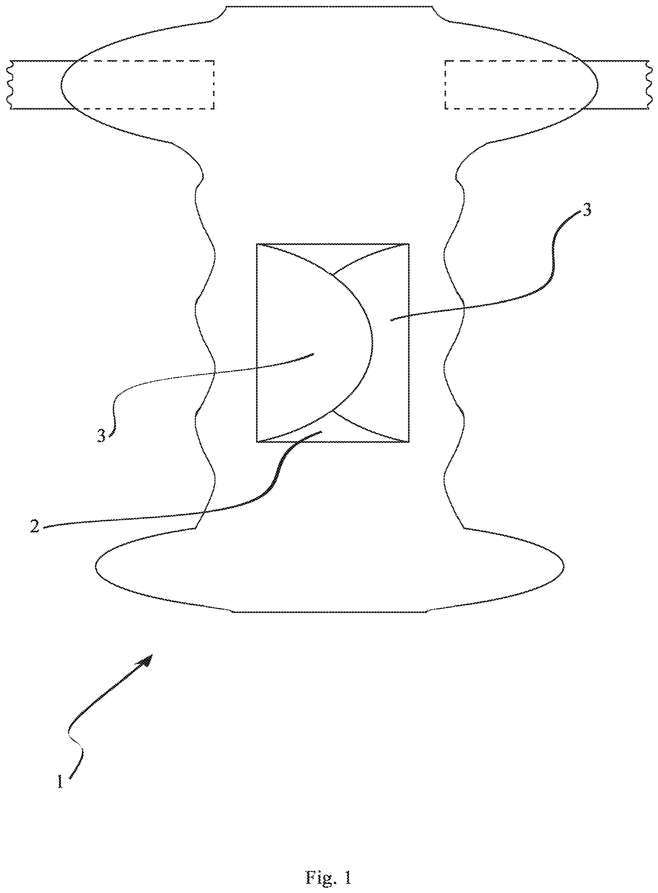

- FIGURE 1 shows a plan view of the absorbent article 1 represented by a diaper. The figure in question shows the presence of the tray-like housing 2 adapted to house the feces of the wearer of said absorbent article. Also observable is the closure system with superimposed tabs 3 with which said housing 2 is equipped. In the embodiments according to the claims, at least one of the tabs (3) is provided, on its edge (3'), with a cord (6) that when pulled from the outside ensures that the tab is positioned in a closed configuration if the entrance of the feces into the tray prevented the repositioning thereof in said closed configuration.

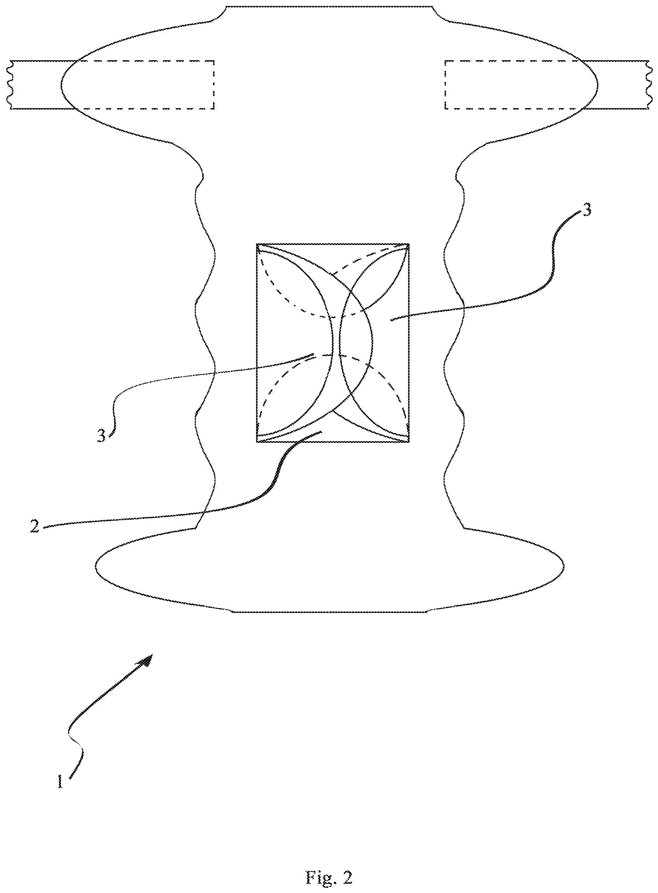

- FIGURE 2 shows a plan view of the absorbent article 1. More in detail the figure in question shows that said absorbent article 1 has a tray-like housing 2 comprising a closure system provided with a plurality of superimposed tabs 3 which have an increasing extension from top to bottom. Such arrangement of the tabs allows ensuring the closure of the housing 2 following the passage of the feces and the isolation of the latter in the tray, so as to prevent the skin of the wearer from contacting the feces, with possible onset of infection risk. In the embodiments according to the claims, at least one of the tabs (3) is provided, on its edge (3'), with a cord (6) that when pulled from the outside ensures that the tab is positioned in a closed configuration if the entrance of the feces into the tray prevented the repositioning thereof in said closed configuration.

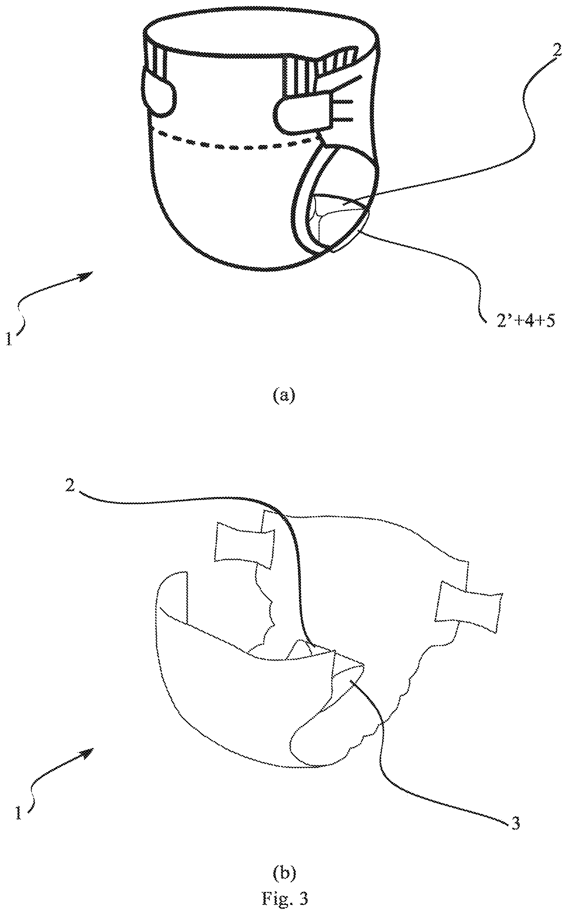

- FIGURE 3 shows a perspective view of the absorbent article 1 in closed configuration, i.e. when worn (fig. 3(a)) and in open configuration (fig.3(b)). The figures in question in particular intend to indicate the presence of the clay-based composition 5 within the tray-like housing 2 and the presence of the film 4, made of biocompatible material, adapted to cover said composition which coats the internal surfaces 2' of said housing and which is such to be dissolved when it is in contact with the moist fraction of the entering feces.

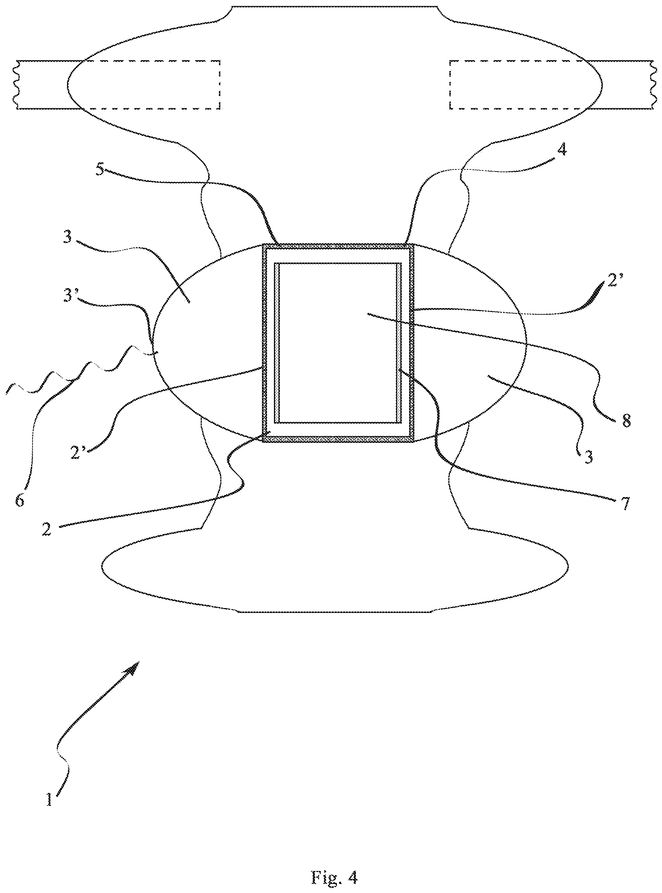

- FIGURE 4 shows a plan view of the absorbent article 1 according to the present invention. More in detail the figure in question shows that said absorbent article can comprise a cord 6 that can be found on at least one of the tabs 3 at the height of its edge 3', adapted to ensure, when tensioned, the closure of the housing 2 following the entrance therein of the feces of the diaper wearer. Also indicated is the presence of sensors 7 for detecting occult blood, pH, salinity, urea, glucose, and an absorbent fluff 8, which can be found on the bottom of the tray-like housing 2, specific for the absorption of excess liquids which reach it via percolation.

### Detailed description of the invention

In all its embodiments, the object of the present description is an absorbent article 1, by way of a non-limiting example a diaper for children, which in addition to offering all the advantages already offered by the commercially-available diapers in ergonomic terms and for capacity of absorbing the physiological liquids, typically urine, is also able to protect the skin from the risk of onset of infections determined by bacterial proliferation, caused by the contact of the skin with the feces possibly housed in the diaper.

More in detail the absorbent article 1 according to the present invention is capable of isolating and compacting, in its structure, the possibly present feces, also neutralizing the potential bacterial action thereof, causing the possibility of onset of infection risk. All of this by preventing the feces from constantly contacting the skin of the wearer, typically of the child, up until the diaper is changed.

Still more in detail, the present absorbent article 1 is characterized by the presence in its structure of a particular combination of a known substance with compacting and neutralizing action against the feces, with a structural characteristic of the present absorbent article itself. Specifically, said absorbent article 1 is characterized in all embodiments thereof in that it has, in its structure, a preferably self-expandable tray-like housing 2 in which a clay-based composition is present, coated with a suitable film made of biopolymer material which is dissolved in contact with the feces at the inlet into said tray, allowing the exit of said composition which can thus carry out its neutralizing action within the tray in which the feces, being neutralized, remain isolated.

More specifically, the absorbent article 1 in its preferred embodiment is a diaper for children which, in addition to having the structure and absorbing properties common to the latest commercially-available diapers, comprises in its structure, at the height of its central portion, a self-expandable tray-like housing 2 provided on the upper part with a system with superimposed tabs 3 which allows the entrance of the feces via gravity within said housing 2. The latter has, on its internal walls 2', a film 4 of biocompatible material adapted to contain a pre-established quantity of a clay-based composition 5 which coats said internal walls 2' of said housing 2. Such composition, thickening and clay-based, has variable grain size and further comprises thickening polymers. Said film 4 made of biocompatible material can be made of polyvinyl alcohol, polyacrylic acid or salts thereof, alginic acid, sodium alginate, calcium alginate or other alginate salts or similar substances capable of being dissolved in contact with the feces or with other substantially moist products. In particular, solid feces, with their liquid component, once within the tray come into contact with and are covered by the film 4 of biocompatible polymer material which is dissolved on their surface.

Once said film 4 has been dissolved, the feces come in contact with the clay composition 5 which, by mechanical action due to the effect of gravity, is mixed with the feces present in the tray, causing the thickening and the hardening thereof. The closure of the tabs occurs automatically following the passage of the feces into the tray, thus ensuring that the skin of the wearer is protected against the risk of contacting the feces.

As repeated multiple times during in the course of the present description, the absorbent article 1 according to the present invention allows: thickening and hardening the feces and neutralizing the bacterial action thereof, and preventing the direct contact of the feces with the skin of the wearer, since once present the feces are confined and isolated within the tray-like housing 2.

In order to optimize the closure mechanism of the system of superimposed tabs 3 from which the feces enter into the tray, the embodiments according to the present invention provide that the absorbent article 1 is provided with a tray-like housing 2 with a closure system with superimposed tabs in which at least one of said tabs 3 is provided on its edge 3' with a cord 6 that, when pulled from the outside, ensures that the tab is positioned in closed configuration, if the entrance of the feces into the tray prevented the repositioning thereof in said closed configuration.

For the same purpose, a further embodiment according to the present invention provides that said absorbent article 1 is provided with a tray-like housing 2 comprising a closure system provided with a plurality of superimposed tabs 3 having a gradual extension increasing from top to bottom. Such embodiment allows said tabs and in particular the upper tabs, i.e. those directly in contact with the skin of the wearer, having a smaller extension with respect to those of the underlying tabs, following the passage of the feces, to be spontaneously repositioned in their initial configuration, with a movement that otherwise tends to decrease as the extension of said lower tabs increases. All this with the result of ensuring that the closure of the tray-like housing 2 is assured, due to at least the closure of the first tabs 3 which are situated directly in contact with the skin. It follows that the feces remain isolated in the tray.

Further embodiments according to the present invention provide that, independent of the number, profile and spatial arrangement of the tabs 3, the latter are made - at least on their upper surface, or on the surface directed towards the skin of the wearer - of a material with little porosity, which instead has a sufficiently smooth surface preferably covered with an oily biocompatible substance that facilitates via sliding the entrance of the feces into the tray, without the latter being able to contaminate the surface of the same tabs and consequently the skin of the wearer who is in contact therewith.

It is also in the interest of the Applicant to specify that the composition 5 present in the absorbent article 1 according to the present invention can, by way of a non-limiting example, comprise: silica; aluminum; calcium; potassium iron; magnesium; sodium; manganese; phosphorus. Preferably said composition 5 comprises: silica at 49.6% by weight; calcium at 8% by weight; iron at 5% by weight; potassium at 4.0% by weight; magnesium at 2.4% by weight; sodium at 0.2% by weight; manganese at 0.2% by weight; phosphorus at 0.14% by weight.

The film coating said composition, self-dissolving following its contact with the feces at the inlet into the tray-like housing 2, is obtained by way of a non-limiting example by dissolving, in water, polyvinyl alcohol with molecular weight equal to 100,000 at a concentration of 10% by weight. The solution is stirred up to obtaining a uniform solution, poured within a container with flat base and dehydrated at the temperature of 40°C in the presence of desiccant salt.

The final dehydrated film thus obtained, detached from its support, can then be easily connected, by way of a non-limiting example by means of sewing, on the internal walls 2' of the housing.

In all embodiments thereof, the clay composition of the absorbent article 1 according to the present invention has variable grain size with one part fine, even micrometric, for a greater aggregating power and one part coarser, even on the order of a millimeter, in order to facilitate the dispersion of the same within the feces. The total weight of said composition is, by way of a non-limiting example, comprised between 150 g and 350 g. Typically for diapers usable by children, the content of said composition varies from 150 g to 200 g while for those usable by elderly people, or in general by adults with problems of incontinence, the content of said composition varies from 300 g to 350 g.

Further embodiments according to the present invention provide that, within the tray-like housing 2, there is at least one sensor 7 for detecting occult blood, pH, salinity, urea, glucose. On the bottom of the same tray, an absorbent fluff 8 specific for the absorption of excess liquids, which reach it via percolation, can also be present in this embodiment as in all the embodiments according to the present invention.

## Claims

1. Absorbent article (1) adapted for absorbing and housing human excrements and urine, said absorbent article (1) being wearable by an incontinent subject, said absorbent article (1) being **characterized in that** it comprises, in its structure at the height of its central portion, a composition (5) with variable grain size with clay and thickening polymer base, said composition (5) compacting and neutralizing the bacterial action of the feces, said absorbent article (1) also having a self-expandable tray-like housing (2) that is adapted to house the feces, said composition (5) able to be found on the internal surfaces (2') of said housing (2) and being coated with a suitable film (4) of biocompatible material capable of being dissolved in contact with the feces at the inlet into said housing (2), said composition (5) compacting and neutralizing the feces present in said housing (2), the latter also being provided with a closure system (3) with superimposed tabs which allows the entrance of the feces via gravity within said housing (2) and **wherein** at least one of the tabs (3) is provided, on its edge (3'), with a cord (6) that when pulled from the outside ensures that the tab is positioned in a closed configuration if the entrance of the feces into the tray prevented the repositioning thereof in said closed configuration and **wherein** the clay-based composition (5) has variable grain size with one part fine and micrometric, and one part coarser, on the order of a millimeter.

2. Absorbent article (1) according to the preceding claim **wherein** said film (4) of biocompatible material, capable of being dissolved when it is in contact with the feces, is attained with a substance selected from the group constituted by: polyvinyl alcohol, polyacrylic acid or salts thereof, alginic acid, sodium alginate, calcium alginate or other alginate salts capable of being dissolved in contact with the feces or with other moist products, and wherein said clay-based composition (5) comprises silica; aluminum; calcium; potassium; iron; magnesium; sodium; manganese; phosphorus.

3. Absorbent article (1) according to the preceding claim **wherein** the composition (5) with clay base comprises silica at 49.6% by weight; calcium at 8% by weight; iron at 5% by weight; potassium at 4.0% by weight; magnesium at 2.4% by weight; sodium at 0.2% by weight; manganese at 0.2% by weight; phosphorus at 0.14% by weight.

4. Absorbent article (1) according to any one of the preceding claims **wherein** the tray-like housing (2) is provided with a closure system comprising a plurality of superimposed tabs (3) having a gradual extension increasing from top to bottom, said configuration of the tabs allowing said tabs and those above, i.e. those directly in contact with the skin of the wearer and having a smaller extension than those of the underlying tabs, to be, following the passage of the feces, spontaneously repositioned into their initial closed configuration, ensuring the closure of said tray-like housing (2) due to at least the closure of the first tabs (3) which are situated directly in contact with the skin and ensuring the isolation of the feces in the tray.

5. Absorbent article (1) according to any one of the preceding claims **wherein** the tabs (3) have, on at least their surface directed towards the skin of the wearer of said absorbent article (1), an oily and biocompatible substance which facilitates the entrance of the feces into the tray via sliding, without the feces being able to contaminate the surface of the same tabs and consequently the skin of the wearer who comes into contact therewith.

6. Absorbent article (1) according to any one of the preceding claims **wherein** within the tray-like housing (2), at least one sensor (7) is present for detecting occult blood, pH, salinity, urea, glucose.

7. Absorbent article (1) according to any one of the preceding claims **wherein** on the bottom of the tray-like housing (2), an absorbent fluff (8) is also present which is specific for the absorption of excess liquids which reach it via percolation.

8. Absorbent article (1) according to any one of the preceding claims **wherein** the clay-based composition (5) has variable grain size with one part fine and micrometric, for a greater feces aggregating and compacting power, and one part coarser, on the order of a millimeter, in order to facilitate the dispersion of the same within the feces, said composition (5) having an overall weight comprised between 150 g and 350 g, said composition being coated with the film (4) connected by means of sewing to the internal surfaces (2') of said housing (2).

9. Absorbent article (1) according to the preceding claim **wherein** the content of the clay-based composition (5) varies from 150 g to 200 g, said absorbent article (1) being a diaper for children.

10. Absorbent article (1) according to claim 9 **wherein** the content of the clay-based composition (5) varies from 300 g to 350 g, said absorbent article (1) being a diaper for elderly people or incontinent adults.

## Patentansprüche

1. Absorbierender Artikel (1), der zum Absorbieren und Aufnehmen von menschlichen Exkrementen und Urin geeignet ist, wobei der absorbierende Artikel (1) von einer inkontinenten Person getragen werden kann, wobei der absorbierende Artikel (1) **dadurch gekennzeichnet ist, dass** er in seiner Struktur auf Höhe seines mittleren Abschnitts eine Zusammensetzung (5) mit unterschiedlicher Korngröße auf Ton- und Verdickungspolymer-Basis aufweist, wobei die Zusammensetzung (5) die bakterielle Wirkung der Fäkalien verdichtet und neutralisiert, wobei der absorbierende Artikel (1) außerdem ein selbstexpandierendes, schalenartiges Gehäuse (2) aufweist, das dazu geeignet ist, die Fäkalien aufzunehmen, wobei die Zusammensetzung (5) auf den Innenflächen (2') des Gehäuses (2) gefunden werden kann und mit einer geeigneten Folie (4) aus biokompatiblem Material beschichtet ist, das sich bei Kontakt mit den Fäkalien am Einlass in das Gehäuse (2) aufgelöst werden kann, wobei die Zusammensetzung (5) die in dem Gehäuse (2) vorhandenen Fäkalien verdichtet und neutralisiert, wobei letzteres außerdem mit einem Verschlusssystem (3) mit übereinanderliegenden Laschen versehen ist, das den Eintritt der Fäkalien durch Schwerkraft in das Gehäuse (2) ermöglicht, und **wobei** zumindest eine der Laschen (3) an ihrem Rand (3 ') mit einer Schnur (6) versehen ist, die, wenn sie von außen gezogen wird, dafür sorgt, dass die Lasche in einer geschlossenen Konfiguration positioniert wird, wenn der Eintritt der Fäkalien in die Schale das Rückstellen derselben in die geschlossene Konfiguration verhindert hat, und **wobei** die auf Ton basierende Zusammensetzung (5) eine unterschiedliche Korngröße aufweist, wobei ein Teil fein und mikrometrisch und ein Teil gröber in der Größenordnung von einem Millimeter ist.

2. Absorbierender Artikel (1) gemäß dem vorstehenden Anspruch, **wobei** die Folie (4) aus biokompatiblem Material, die sich auflösen kann, wenn sie mit den Fäkalien in Kontakt steht, mit einer Substanz erreicht wird, die ausgewählt ist aus der Gruppe, die gebildet ist durch: Polyvinylalkohol, Polyacrylsäure oder deren Salzen, Alginsäure, Natriumalginat, Calciumalginat oder anderen Alginatsalzen, die sich bei Kontakt mit den Fäkalien oder mit anderen feuchten Produkten auflösen können, und wobei die auf Ton basierende Zusammensetzung (5) Siliziumdioxid; Aluminium; Kalzium; Kalium; Eisen; Magnesium; Natrium; Mangan; Phosphor aufweist.

3. Absorbierender Artikel (1) gemäß dem vorstehenden Anspruch, **wobei** die Zusammensetzung (5) mit Tonbasis Siliziumdioxid mit 49,6 Gew.-%; Kalzium mit 8 Gew.-%; Eisen mit 5 Gew.-%; Kalium mit 4,0 Gew.-%; Magnesium mit 2,4 Gew.-%; Natrium mit 0,2 Gew.-%; Mangan mit 0,2 Gew.-%; Phosphor mit 0,14 Gew.-% aufweist.

4. Absorbierender Artikel **(1)** gemäß einem der vorstehenden Ansprüche, **wobei** das schalenartige Gehäuse (2) mit einem Verschlusssystem versehen ist, das mehrere übereinanderliegende Laschen (3) aufweist, die sich von oben nach unten allmählich verlängern, wobei die Konfiguration der Laschen es ermöglicht, dass sich die Laschen und diejenigen darüber, d. h. die direkt mit der Haut des Trägers in Kontakt stehen und eine geringere Ausdehnung als diejenigen der darunter liegenden Laschen aufweisen, nach dem Durchgang der Fäkalien spontan in ihre ursprüngliche geschlossene Konfiguration zurückgebracht werden, wodurch der Verschluss des schalenartigen Gehäuses (2) aufgrund zumindest des Verschlusses der ersten Laschen (3), die sich in direktem Kontakt mit der Haut befinden, sichergestellt und die Isolierung der Fäkalien in der Schale sicherstellen, gewährleistet wird.

5. Absorbierender Artikel (1) gemäß einem der vorstehenden Ansprüche, **wobei** die Laschen (3) zumindest auf ihrer zur Haut des Trägers des absorbierenden Artikels (1) gerichteten Oberfläche eine ölige und biokompatible Substanz aufweisen, die den Eintritt der Fäkalien in die Schale durch Gleiten erleichtert, ohne dass die Fäkalien die Oberfläche derselben Laschen und folglich die Haut des Trägers, der mit ihnen in Kontakt kommt, verunreinigen kann.

6. Absorbierender Artikel (1) nach einem der vorstehenden Ansprüche, **wobei** innerhalb des schalenartigen Gehäuses (2) zumindest ein Sensor (7) zum Ermitteln von okkultem Blut, pH, Salzgehalt, Harnstoff, Glukose vorhanden ist.

7. Absorbierender Artikel (1) gemäß einem der vorstehenden Ansprüche, **wobei** auf dem Boden des schalenartigen Gehäuses (2) auch eine absorbierende Flockung (8) vorhanden ist, die speziell für die Absorption von überschüssigen Flüssigkeiten vorgesehen ist, die sie durch Versickern erreichen.

8. Absorbierender Artikel (1) nach einem der vorstehenden Ansprüche, **wobei** die auf Ton basierende Zusammensetzung (5) eine unterschiedliche Korngröße aufweist, wobei ein Teil für eine größere Fäkalienaggregations- und -verdichtungsleistung fein und mikrometrisch ist, und ein Teil gröber ist, in der Größenordnung von einem Millimeter, um die Dispersion derselben innerhalb der Fäkalien zu erleichtern, wobei die Zusammensetzung (5) ein Gesamtgewicht zwischen 150 g und 350 g aufweist, wobei die Zusammensetzung (5) mit der Folie (4), die durch Nähen an die Innenflächen (2') des Gehäuses (2) verbunden ist, beschichtet ist.

9. Absorbierender Artikel **(1)** gemäß dem vorstehenden Anspruch, **wobei** der Gehalt der auf Ton basierenden Zusammensetzung (5) zwischen 150 g und 200 g variiert, wobei der absorbierende Artikel (1) eine Windel für Kinder ist.

10. Absorbierender Artikel **(1)** gemäß Anspruch 9, **wobei** der Gehalt der auf Ton basierenden Zusammensetzung (5) zwischen 300 g und 350 g variiert, wobei der absorbierende Artikel (1) eine Windel für ältere Menschen oder inkontinente Erwachsene ist.

## Revendications

1. Article absorbant (1) adapté pour absorber et loger des excréments et de l'urine humains, ledit article absorbant (1) pouvant être porté par un sujet incontinent, ledit article absorbant (1) étant **caractérisé en ce qu'**il comprend, dans sa structure à la hauteur de sa partie centrale, une composition (5) à taille de grain variable à base d'argile et de polymère épaississant, ladite composition (5) compactant et neutralisant l'action bactérienne des selles, ledit article absorbant (1) ayant également un logement (2) en forme de plateau auto-expansible qui est adapté pour loger les selles, ladite composition (5) pouvant se trouver sur les surfaces internes (2') dudit logement (2) et étant revêtue d'un film approprié (4) de matériel biocompatible capable d'être dissous au contact des selles au niveau de l'entrée dans ledit logement (2), ladite composition (5) compactant et neutralisant les selles présentes dans ledit logement (2), ce dernier étant également doté d'un système de fermeture (3) avec des languettes superposées qui permettent l'introduction des selles par gravité à l'intérieur dudit logement (2) et dans **lequel** au moins l'une des languettes (3) est dotée, sur son bord (3'), d'un cordon (6) qui, lorsqu'il est tiré de l'extérieur, assure que la languette est positionnée dans une configuration fermée si l'introduction de selles dans le plateau a empêché le repositionnement de celle-ci dans ladite configuration fermée et dans **lequel** la composition à base d'argile (5) présente une taille de grain variable avec une partie fine et micrométrique, et une partie plus grossière, de l'ordre d'un millimètre.

2. Article absorbant (1) selon la revendication précédente dans **lequel** ledit film (4) de matériel biocompatible, capable d'être dissous lorsqu'il est en contact avec les selles, est obtenu à partir d'une substance sélectionnée parmi le groupe constitué de : alcool polyvinylique, acide polyacrylique ou sels de ceux-ci, acide alginique, alginate de sodium, alginate de calcium ou autres sels d'alginate capables d'être dissous au contact des selles ou d'autres produits humides, et dans lequel ladite composition à base d'argile (5) comprend de la silice ; aluminium ; calcium ; potassium ; fer ; magnésium ; sodium ; manganèse ; phosphore.

3. Article absorbant (1) selon la revendication précédente dans **lequel** la composition (5) à base d'argile comprend de la silice à 49,6 % en poids ; calcium à 8 % en poids ; fer à 5 % en poids ; potassium à 4,0 % en poids ; magnésium à 2,4 % en poids ; sodium à 0,2 % en poids ; manganèse à 0,2 % en poids ; phosphore à 0,14 % en poids.

4. Article absorbant (1) selon l'une des revendications précédentes dans **lequel** le logement (2) de type plateau est doté d'un système de fermeture comprenant une pluralité de languettes superposées (3) présentant une extension progressive croissante de haut en bas, ladite configuration des languettes permettant auxdites languettes et à celles au-dessus, c'est-à-dire celles en contact direct avec la peau du porteur et présentant une extension plus petite que celles des languettes sous-jacentes, d'être, à la suite du passage des selles, repositionnées spontanément dans leur configuration fermée initiale, assurant la fermeture dudit logement (2) de type plateau du fait au moins de la fermeture des premières languettes (3) qui sont situées en contact direct avec la peau et assurant l'isolation des selles dans le plateau.

5. Article absorbant (1) selon l'une des revendications précédentes dans **lequel** les languettes (3) présentent, sur au moins leur surface dirigée vers la peau du porteur dudit article absorbant (1), une substance huileuse et biocompatible qui facilite l'introduction des selles dans le plateau par glissement, sans que les selles ne soient capables de contaminer la surface des mêmes languettes et par conséquent la peau du porteur qui entre en contact avec celles-ci.

6. Article absorbant (1) selon l'une des revendications précédentes, dans **lequel,** à l'intérieur du logement (2) de type plateau, au moins un capteur (7) est présent pour détecter du sang occulte, pH, salinité, urée et glucose.

7. Article absorbant (1) selon l'une des revendications précédentes, dans **lequel,** est également présent, sur le fond du logement (2) de type plateau, un duvet absorbant (8) qui est spécifique à l'absorption de liquides excédentaires qui l'atteignent via percolation.

8. Article absorbant (1) selon l'une des revendications précédentes dans **lequel** la composition (5) à base d'argile présente une taille de grain variable avec une partie fine et micrométrique, pour un pouvoir d'agrégation et de compactage de selles plus important, et une partie plus grossière, de l'ordre d'un millimètre, afin de faciliter la dispersion de celle-ci dans les selles, ladite composition (5) ayant un poids global compris entre 150 g et 350 g, ladite composition étant revêtue du film (4) relié au moyen d'une couture aux surfaces internes (2') dudit logement (2).

9. Article absorbant (1) selon la revendication précédente dans **lequel** la teneur de la composition (5) à base d'argile varie de 150 g à 200 g, ledit article absorbant (1) étant une couche pour enfants.

10. Article absorbant (1) selon la revendication 9 dans **lequel** la teneur de la composition (5) à base d'argile varie de 300 g à 350 g, ledit article absorbant (1) étant une couche pour personnes âgées ou adultes incontinents.
